# EUROPEAN PATENT SPECIFICATION

(11) **EP 2 874 591 B1**
(45) Date of publication and mention of the grant of the patent: **18.05.2016**
(21) Application number: 13739776.6
(22) Date of filing: 03.06.2013
(51) Int. Cl.: A61G 13/00, A61G 13/08

(54) **SENSORIZED POSTURAL COUCH FOR PHYSICAL THERAPY**
SENSORISIERTE HALTUNGSLIEGE FÜR PHYSIOTHERAPIE
TABLE D'AUSCULTATION POSTURALE MUNIE DE CAPTEURS POUR THÉRAPIE PHYSIQUE

(30) Priority: 18.07.2012 IT BG20120037
(43) Date of publication of application: 27.05.2015
(73) Proprietor: Tecnobody S.r.l., 24044 Dalmine (BG) (IT)
(72) Inventor: MARCANDELLI, Stefano, I-24040 Stezzano (BG) (IT); CARMINATI, Alessandro, I-24040 Stezzano (BG) (IT)
(74) Representative: Gatti, Enrico
(86) International application number: PCT/IB2013/001130
(87) International publication number: WO 2014/013302

(56) References cited:
- WO-A1-2005/034827
- US-A- 5 774 915
- US-A1- 2006 123 546
- US-A1- 2008 167 684
- US-B1- 6 971 997

## Description

The present Invention relates to a sensorized postural couch of variable geometry for physical therapy, and a method for verifying the muscular tension of a patient before and after patient treatment.

Couches or benches for physical therapy are known, often modular and positionable to enable the therapist to correctly position the patient and facilitate therapeutic operations thereon.

Articulated couches are also known, provided with means for manually or electrically moving one or more of their parts. Examples of couches can be found in US2006/123546, WO2005/034827, US6971997 and US5774915.

An object of the present invention is to provide a sensorized couch of variable geometry for physical therapy which enables the operator to comprehend the structure and posture of the patient.

Another object is to provide a couch which can give an evaluation of the anatomical-functional tensions and/or rotations in the three main planes of the articular body movement (sagittal, transverse and frontal).

A further object is to measure the tensions of all anatomical districts to obtain a single indicator able to give the exact tension exerted on all rear chains (muscular, myofascial).

Another object is to have a system able not only to make postural measurements, but also to display post-evaluation physiotherapeutic treatment.

These and further objects are attained according to the present invention by a sensorized couch of variable geometry for physical therapy comprising: a base; a patient support comprising at least two panels; each panel of said at least two panels being pivoted to each panel adjacent to it; characterised in that each panel of said at least two panels comprises at least three load sensors and an accelerometer.

These objects are also attained by a method for evaluating the muscular tension of a patient, comprising the steps of measuring first loads applied by the patient's body when in the extended supine position on a couch in its flat position, by sensors applied to said couch; measuring second loads applied by the patient's body when in the extended position on said couch in its position inclined by a predetermined angle, by sensors applied to said couch; calculating a first index of muscular tension of the patient's body as the difference between said first and second loads.

Further characteristics of the invention are described in the independent claims.

This solution has various advantages compared with the solutions of the known art.

The physical therapy couch, according to the present invention, for manual treatment and postural evaluation, has a variable geometry and is provided with a network of electronic sensors controlled by a computer.

The particular variable geometry together with the load and inclination measuring devices enable acquisition of the forces applied passively by the patient's body and the pressures exerted actively by the therapist during treatment.

The postural couch is composed of four independent sections which determine the load exerted by the body in the specific body districts, namely: lower limbs, pelvis, lumbar trunk, scapular trunk, and head.

Each individual section is able to determine the load exerted and its position on the panel, similarly to the Centre of Pressure (CoP) of stabilometric systems.

The angles configurable by the support panels of the postural couch ensure subject positioning under predefined and controlled stretching conditions.

This technological solution fully satisfies the requirement of monitoring the tensions and the rotations of the rear chain in the three main planes of the articulated body movement (sagittal, transverse and frontal), hence determining the load variation of the tensions on the entire support surface.

The new system moreover completely perceives all parts of the supported body. This enables a single indicator to be obtained able to give the exact tension of all the rear chains (muscular and myofascial).

The characteristics and advantages of the present invention will be apparent from the ensuing detailed description of a preferred embodiment thereof, illustrated by way of non-limiting example in the accompanying drawings, in which:

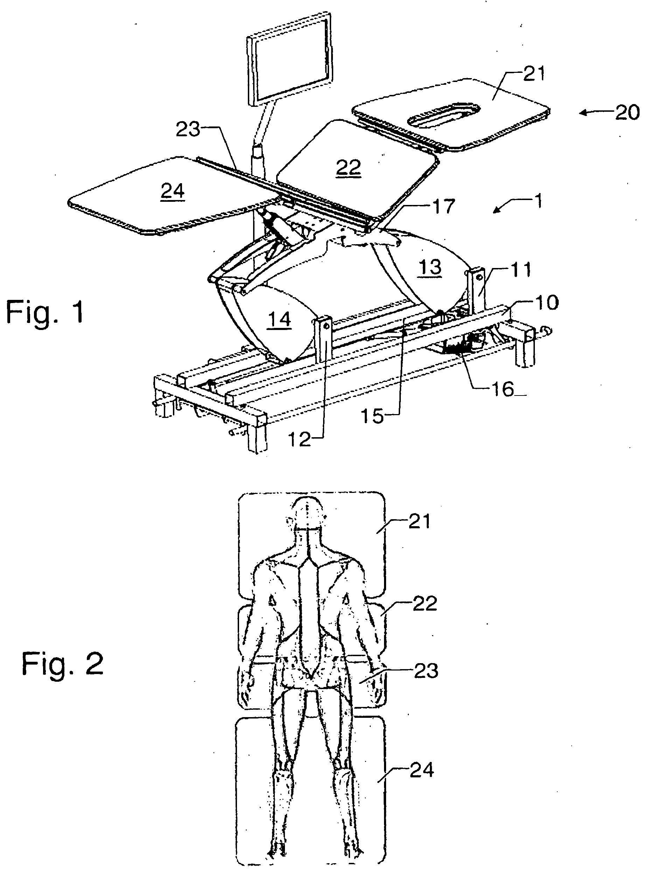

Figure 1 shows a perspective view of a couch of variable geometry for physical therapy, in accordance with the present invention;
Figure 2 shows a design of a human figure with reference to the support surfaces of the sensorized couch of variable geometry for physical therapy, in accordance with the present invention;

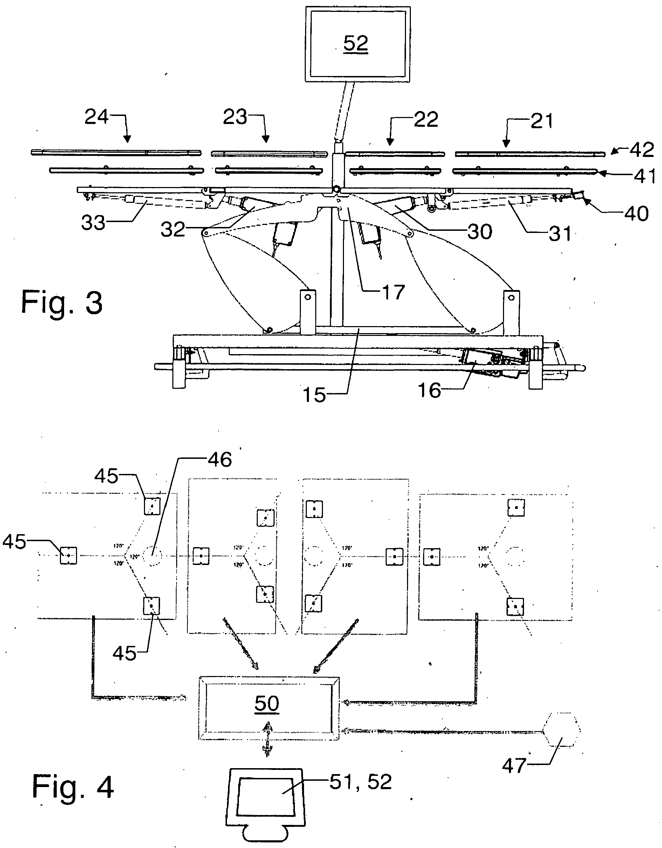

Figure 3 shows a front view of a sensorized couch of variable geometry for physical therapy, partly broken down into its elements, in accordance with the present invention;
Figure 4 shows a block scheme of the sensors positioned on the couch of variable geometry for physical therapy, and on the control means associated therewith, in accordance with the present invention;
Figure 5 shows an example of image representation of a computer connected to a couch of variable geometry for physical therapy, in accordance with the present invention.

With reference to the accompanying figures, a sensorized couch 1 of variable geometry for physical therapy according to the present invention comprises a base 10 on which the couch 1 rests.

The base is a foot-supported tubular structure from which two pairs of vertical bars 11 and 12 rise. The pair of bars 11 is positioned at one end of the base 10 and the pair of bars 12 is positioned at about 2/3 from the first pair of bars 11.

A corner of the base of a triangular structure 13 and 14 is pivoted to each of the vertical bars 11 and 12.

The other corner of the base of each of the triangular structures 13 and 14 is pivoted to a horizontal bar 15.

The bar 15 is fixed, approximately at its centre, to one end of a controlled linear actuator 16. The other end of the linear actuator 16 is fixed to one end of the base 10, namely that at which the pair of vertical bars 11 is present, it being fixed below them.

The vertex of each of the triangular structures 13 and 14 is pivoted to a support 17 of the couch 1, substantial at its central point. The couch 1 is pivoted on the central upper summit of the support 17.

To raise the couch 1 the linear actuator 16 is operated, which by thrusting the bar 15 raises the triangular structures 13 and 14 which by forcing the support 17 upwards raises the couch 1. By operating the linear actuator 16 in the reverse direction the couch descends.

If the couch is totally lowered, the triangular structures 13 and 14 are partially concealed within the base 10.

In the embodiment shown in the figures, the support surface 20 for the patient comprises four panels connected together but independent in terms of angularity. The panel 21 is that on which the scapular trunk and head rest. The panel 22, is that on which the lumbar trunk rests. The panel 23 is that on which the pelvis and femoral part of the leg rest. The pannel 24 is that on which the lower limbs (tibial part) rest.

In a more simplified embodiment, the support surface 20 for the patient can have a smaller number of support panels, for example two in number: one for the upper trunk and one for the lower trunk.

The support 17 is pivoted to the support surface 20, and in particular is pivoted to the two most inner sides of the panels 22 and 23.

The width of the panels 21-24 is substantially the same. The length of the panels 21-24 varies, depending on the length of the aforedefined human body portions. In particular the panel 24 is the longest, the panel 21 is of length slightly less than the panel 24, and the two panels 22 and 23 are still shorter but of substantially the same length.

That side of the panel 22 opposite the side pivoted to the support 17 is pivoted to one end of a controlled linear actuator 30, the other end of which is pivoted on a side of the arc (right in the figure) of the support 17.

The most outer side of the panel 21 is fixed to one end of a manually operated piston 31, the other end of which is pivoted to the outer side of the panel 22.

That side of the panel 23 opposite the side pivoted to the support 17 is pivoted to one end of a controlled linear actuator 32, the other end of which is pivoted on a side of the arc (left in the figure) of the support 17.

The most outer side of the panel 24 is fixed to one end of a manually operated piston 33, the other end of which is pivoted to the outer side of the panel 23.

The controlled linear actuators 16 and 30 used in the present invention are preferably electrical, and when commanded drive forwards and rearwards a rod which emerges from the motor itself.

Alternatively other types of linear actuators can be used such as mechanical, hydraulic, pneumatic.

The linear actuator 16 and the dimensions of the structure formed by the elements 13, 14, 15 and 17 enable the couch to be raised from the floor by an overall distance from 40 cm to 120 cm.

The inclination of the lumbar support panel 22 (and of the scapular panel 21 connected to it) can be varied by the linear actuator 30 from -10° to +55° about a horizontal axis.

The inclination of the pelvis support panel 23 (and of the lower limb panel 24 connected to it) can be varied by the linear actuator 32 from -10° to +45° about a horizontal axis

The inclination of the scapular support panel 21 can be varied by the manually operated piston 31 from -40° to +45° about the panel 22.

The inclination of the leg/foot support panel 24 can be varied by the manually operated piston 33 from -40° to +45° about the panel 23.

Each of the panels 21-24 comprises three layers.

An underlying first mechanical support layer 40 for the panels 21-24, an intermediate second layer 41 including sensors, and an upper finishing layer 42 with appearance and protection functions.

In order to measure the loads and relative positions of the patient, three load sensors 45 and a monoaxial accelerometer 46 are positioned in the layer 41 of each panel 21-24.

In particular, the three load sensors 45 of each panel 21-24 are positioned equidistant at 120° apart, directed along the longitudinal axis with one of their joining axes positioned along the longitudinal axis of the support surface 20.

The accelerometer 46 is preferably positioned, on each panel, with its axis coaxial with that axis joining two load sensors 45 which is parallel to one side of the panel. It is used principally as an inclinometer for the various panels.

A further fluctuating inertial sensor 47 is preferably provided freely positionable for angular kinesthetic measurements.

The individual load sensors 45 have a maximum capacity of 109 kg, a non-linearity of ≤ 0.04% and a resolution equal to 25 g.

The accelerometers 46 have a resolution level of 0.2 degrees within a range of +90° to -90°, such as to enable the inclinations of each panel to be measured with sufficient precision.

The inertial sensor, used for angular degree measurements, is a sensor provided with a gyroscope, accelerometer and triaxial magnetometer.

The main uses of the inertial sensors integrated with the postural couch are basically two in number: kinesiological evaluation pre and post treatment and real time monitoring of the articular district during manipulative treatment (e.g. extremely delicate cervical manipulation). Both the angular evaluations are integrated by the applied load measurements. This signifies that the system determines whether the patient's articulations achieve the same range of motion in specular dx-sx manner, while maintaining the same muscular and myofascial tensions.

The signals of the load sensors 45, of the accelerometers 46 and of the inertial sensors 47, and the commands of the linear actuators 30 and 32, are connected electrically to a concentrator 50 and then to a computer 51, which comprises a monitor 52 positioned on the base 10 (or in the vicinity thereof).

All the sensors are connected to the concentrator 50 which comprises a single electric A/D conversion circuit which consolidates the measured data and communicates with the computer 51 in synchronous mode with frequency configurable from 20Hz to 40Hz.

The computer 51 contains programmes able to obtain the measurements of the various sensors from the concentrator 50, and to calculate the barycentre of the forces of each panel 21-24 by triangulation of the groups of sensors 45.

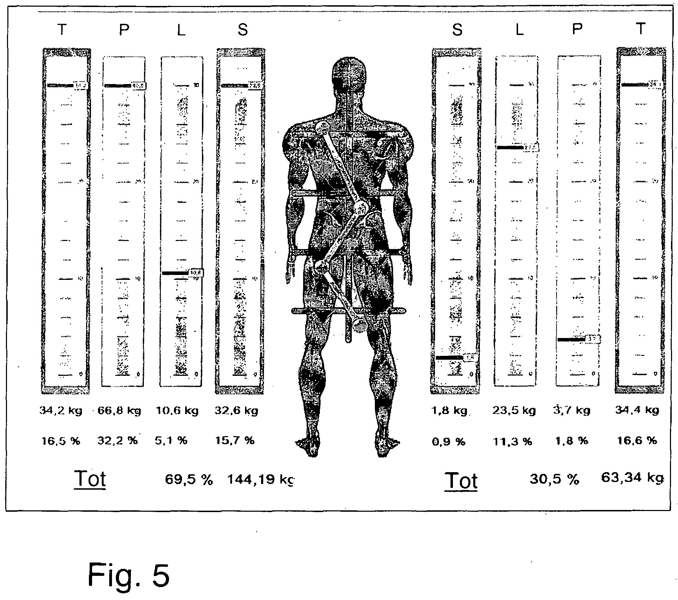

The computer 51 enables all the-physical variables in play during evaluation and treatment to be displayed in real time on the monitor 52, as can be seen for example in Figure 5, which indicates for each panel the loads thereon to the right and left of the linear actuator axis and indicates the position of the barycentre for each panel, where T indicates the lower limb portion, P indicates the pelvis potion, L indicates the lumbar trunk portion, S indicates the scapular and head portion, and Tot indicates the total load, all divided into the right and left part, in percentage and absolute values.

The various loads developed by the subject and the barycentre of each monitored body section (nape-scapular, lumbar, pelvis, legs) are indicated in real time.

The patient can also be subjected to postural training sessions with the aid of visual and acoustic feedback.

Specifically, during the training session the patient is invited by the computer 52 to carry out articular twisting at various levels (scapular, lumbar, pelvis) to attain the proposed targets.

The operation of the device according to the invention is apparent from that described and illustrated, and in particular is substantially as follows.

The subject is placed on the postural couch 1 in a perfectly horizontal supine position (zero position) and while in this position the total weight is measured. Subsequently, the system by changing the configuration puts the subject into a stretching situation by an angle of 120° or 105° or another predetermined angle (evaluation position).

The load measured by the couch panels is the resultant of the patient's weight plus the tension exerted by the rear chain, from which the overall tension index is derived (by subtracting the weight), the various tensions on the separate anatomical segments (nape, scapular, lumbar, pelvis, legs and feet) then being determined. In addition to the value of the tensions, their misalignments (i.e. the positioning of the tension of all muscular chains) are also identified in the four sections into which the body has been divided.

The values and misalignments are displayed on the monitor 52 by suitable graphs.

The couch 1, used to evaluate the tension indexes, is the same as used in the physiotherapeutic treatment. The function of evaluating the muscular tensions and hence of postural evaluation can be carried out simultaneously and on the same system as the treatment function, hence enabling the operator to close the rehabilitative process loop without moving the patient.

By virtue of the intelligent sensoristics with which it is provided, the system inter alia enables the physiotherapeutic work carried out by the operator on the subject to be monitored, hence quantifying and memorizing the intensity of its action and the articular levels applied to the treated anatomical parts by means of integrated sensors.

By observing the computer in real time, the therapist is able to note the intensity of a given manoeuvre in terms of pressure exerted, consequent muscular tension and articular levels applied. In this manner the operator can model his/her intervention by controlled and precise clinical movements.

The entire working session can be filed digitally on the computer 51 for possible future technical re-evaluations and comparison, and to document the entire treatment.

Positioning is then repeated as in the first step (evaluation position) and the current tension exerted by the patient is monitored, hence enabling the operator and patient to evaluate the results obtained by the therapy, by comparing the two measurements.

In other words, the method comprises the following steps:
* measuring the loads applied by the patient's body when in the supine extended position on a flat couch (zero position);
* measuring the loads applied by the patient's body when in the extended position on the couch at a predetermined angle (evaluation position);
* calculating and filing the subject tension index as the difference between the two preceding measurements;

* possible physiotherapeutic and and/or manipulation treatment;
* with real time display of the instantaneous loads applied by the therapist;
* and alternatively to, or in combination with, the physiotherapeutic and manipulation treatment, patient auto-training on the couch with feedback displayed on the monitor;
* measuring the loads applied by the patient's body when in an extended position on the couch at a predetermined angle;
* calculating the current tension index;
* comparing the pre and post treatment indexes;
* filing the data.

With the couch described herein the muscular tension index can be evaluated as aforedescribed and then memorized.

To verify changes in the muscular tension index, after a predetermined time period a further evaluation of the muscular tension index can be carried out and compared with the preceding, and the Variations evaluated.

A sensorized couch of variable geometry for physical therapy is hence made available by which the tension of the patient's rear chain can be verified, with the operator operating on one and the same couch and observing in real time the forces applied, after which the patient's muscular tension can again be verified in order to observe the improvements achieved by the therapy.

All the acquired data can be memorized for subsequent comparison and to maintain the patient's history.

In practice, the materials used for the couch and its dimensions can be chosen at will depending on requirements and on the state of the art.

The couch and method conceived in this manner are susceptible to numerous modifications and variants, all falling within the scope of the inventive concept; moreover all details can be replaced by technically equivalent elements.

## Claims

1. A,sensorized couch (1) of variable geometry for physical therapy, comprising: a base (10); a patient support (17) comprising at least two panels (21-24); each panel of said at least two panels (21-24) being pivoted to each panel adjacent to it; **characterised in that** each panel of said at least two panels (21-24) comprises at least three load sensors (45) and an accelerometer (46).

2. A couch (1) as claimed in claim 1, **characterised in that** said at least two panels (21-24) are four in number.

3. A couch (1) as claimed in claim 1, **characterised by** comprising means (13-14) for vertically moving said patient support (17).

4. A couch (1) as claimed in claim 1, **characterised in that** said at least three load sensors (45) are positioned at 120° apart.

5. A couch (1) as claimed in claim 2 and 3, **characterised in that** the two central panels (22-23) of said four panels (21-24) are pivoted to each other and pivoted to said vertical movement means (13-14).

6. A couch (1) as claimed in claim 5, **characterised by** comprising two linear actuators (16, 30), fixed to said vertical movement means (13-14), which angularly move said two central panels (22-23).

7. A couch (1) as claimed in claim 5, **characterised by** comprising two pistons (31-33), fixed to said two central panels (22-23), which angularly move the peripheral panels (21, 24) of said four panels (21-24).

8. A couch (1) as claimed in claim 1, **characterised by** comprising a computer (51) which receives the signals originating from said load sensors (45) and from said accelerometers (46), and a monitor (52) on which the information received and processed can be displayed.

9. A couch (1) as claimed in claim 1, **characterised by** comprising a fluctuating inertial sensor (47) freely positionable for angular kinesthetic measurements.

## Patentansprüche

1. Mit Sensor versehene Liege (1) variabler Geometrie für die Physiotherapie, die aufweist: eine Basis (10), einen Pati-ententräger (17), der wenigstens zwei Platten (21 - 24) umfasst, wobei jede Platte der wenigstens zwei Platten (21 - 24) schwenkbar mit jeder ihr benachbarten Platte verbunden ist, **dadurch gekennzeichnet, dass** jede Platte der wenigstens zwei Platten (21 - 24) wenigstens drei Kraftsensoren (45) und einen Beschleunigungsmesser (46) aufweist.

2. Liege (1) wie in Anspruch 1 beansprucht, **dadurch gekennzeichnet, dass** die wenigstens zwei Platten (21 - 24) der Anzahl nach vier sind.

3. Liege (1) wie in Anspruch 1 beansprucht, **gekennzeichnet durch** Einrichtungen (13 -14) zum vertikalen Bewegen des Patiententrägers (17).

4. Liege (1) wie in Anspruch 1 beansprucht, **dadurch gekennzeichnet, dass** die wenigstens drei Kraftsensoren (45) 120° auseinander positioniert sind.

5. Liege (1) wie in Anspruch 2 und 3 beansprucht, **dadurch gekennzeichnet, dass** die beiden zentralen Platten (22 - 23) der vier Platten (21 - 24) schwenkbar miteinander und schwenkbar mit den vertikalen Bewegungseinrichtungen (13 - 14) verbunden sind.

6. Liege (1) wie in Anspruch 5 beansprucht, **dadurch gekennzeichnet, dass** zwei lineare Stellglieder (16, 30) vorhanden sind, die an den vertikalen Bewegungseinrichtungen (13 - 14) befestigt sind und die eine Winkelbewegung der zentralen Platten (22 - 23) bewirken.

7. Liege (1) wie in Anspruch 5 beansprucht, **dadurch gekennzeichnet, dass** zwei Kolben (31 - 33) vorhanden sind, die an den beiden zentralen Platten (22 - 23) befestigt sind und die Winkelbewegung der am Rand befindlichen Platten (21, 24) der vier Platten (21 - 24) bewirken.

8. Liege (1) wie in Anspruch 1 beansprucht, **dadurch gekennzeichnet, dass** sie einen Computer (51), der von den Kraftsensoren (45) und dem Beschleunigungssensoren (46) stammende Signale empfängt, und einen Monitor (52) umfasst, auf dem empfangene und verarbeitete Information angezeigt werden kann.

9. Liege (1) wie in Anspruch 1 beansprucht, **dadurch gekennzeichnet, dass** sie einen fluktuierenden Trägheitssensor (47) aufweist, der frei positionierbar ist für winkelmäßige kinästhetische Messungen.

## Revendications

1. Table d'auscultation (1) de géométrie variable pour thérapie physique, comprenant une base (10) ; un support de patient (17) comprenant au moins deux panneaux (21-24) ; chaque panneau desdits au moins deux panneaux (21-24) pouvant pivoter relativement à chaque panneau adjacent à celui-ci ; **caractérisée en ce que** chaque panneau desdits au moins deux panneaux (21-24) comprend au moins trois capteurs de charge (45) et un accéléromètre (46).

2. Table (1) selon la revendication 1, **caractérisée en ce que** lesdits au moins deux panneaux (21-24) sont au nombre de quatre.

3. Table (1) selon la revendication 1, **caractérisée en ce qu'**elle comprend des moyens (13-14) pour déplacer verticalement ledit support de patient (17).

4. Table (1) selon la revendication 1, **caractérisée en ce que** lesdits au moins trois capteurs de charge (45) sont positionnés à 120° d'écart.

5. Table (1) selon les revendications 2 et 3, **caractérisée en ce que** les deux panneaux centraux (22-23) desdits quatre panneaux (21-24) peuvent pivoter relativement l'un à l'autre et peuvent pivoter relativement auxdits moyens de déplacement vertical (13-14).

6. Table (1) selon la revendication 5, **caractérisée en ce qu'**elle comprend deux actionneurs linéaires (16, 30), fixés auxdits moyens de déplacement vertical (13-14), qui déplacent de manière angulaire lesdits deux panneaux centraux (22-23).

7. Table (1) selon la revendication 5, **caractérisée en ce qu'**elle comprend deux pistons (31-33) fixés auxdits deux panneaux centraux (22-23), qui déplacent de manière angulaire lesdits panneaux périphériques (21, 24) desdits quatre panneaux (21-24).

8. Table (1) selon la revendication 1, **caractérisée en ce qu'**elle comprend un ordinateur (51) qui reçoit les signaux provenant desdits capteurs de charge (45) et desdits accéléromètres (46), et un moniteur (52) sur lequel les informations reçues et traitées peuvent être affichées.

9. Table (1) selon la revendication 1, **caractérisée en ce qu'**elle comprend un capteur d'inertie fluctuante (47) positionnable librement pour des mesures angulaires kinesthésiques.
